(19) **Europäisches Patentamt / European Patent Office / Office européen des brevets**

(11) **EP 2 929 853 A2**

(12) # EUROPEAN PATENT APPLICATION

(43) Date of publication:
**14.10.2015 Bulletin 2015/42**

(51) Int Cl.:
***A61B 19/00*** (2006.01)

(21) Application number: **15159476.9**

(22) Date of filing: **17.03.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **18.03.2014 JP 2014054475**

(71) Applicant: **Ricoh Company, Ltd.**
**Tokyo 143-8555 (JP)**

(72) Inventors:
  • **Tatewaki, Tadafumi**
    **Tokyo, 143-8555 (JP)**

  • **Kurisu, Norio**
    **Tokyo, 143-8555 (JP)**
  • **Kawase, Tsutomu**
    **Tokyo, 143-8555 (JP)**
  • **Kutami, Atsushi**
    **Tokyo, 143-8555 (JP)**
  • **Nakamura, Yasuyuki**
    **Tokyo, 143-8555 (JP)**
  • **Nakata, Yoshiko**
    **Tokyo, 143-8555 (JP)**

(74) Representative: **Leeming, John Gerard**
    **J A Kemp**
    **14 South Square**
    **Gray's Inn**
    **London WC1R 5JJ (GB)**

(54) **MEDICAL APPARATUS WITH IC, AND MEDICAL APPARATUS MANAGEMENT SYSTEM**

(57)     Disclosed is a medical apparatus with an IC chip. The medical apparatus includes a housing including a metallic part having a predetermined opening part, and an IC chip module having two conductive members, the conductive members being arranged on respective sides of the IC chip supplied with electricity via the conductive members. The IC chip module is attached such that the conductive members are arranged close to or on respective sides in a width direction of the predetermined opening part of the housing.

FIG.1

EP 2 929 853 A2

**Description**

The disclosures herein relate to a medical apparatus with an IC chip, and a medical apparatus management system.

In hospitals, loss of medical apparatuses or medical materials after surgical operations may lead to medical accidents such as leaving a medical apparatus such as a surgical knife or foreign material inside the body of a patient. There are various types of medical apparatuses including surgical knives, surgical scissors, medical suture needles, and surgical forceps. Hence, to prevent medical accidents, staff members (humans) in the hospitals generally manage such medical apparatuses by counting the number of medical apparatuses. For example, the staff members may count the number of medical apparatuses that come in or go out of an operating room before and after an operation. Or the staff members may compare the number of medical apparatuses prepared before the operation with the number of medical apparatuses to be cleaned after the operation. Further, X-rays or CT scans may be used to determine the presence or absence of remnants and the like in the body at the end of a surgical operation (often after surgical wound closure or sealing).

However, in such a management method, the counted number may be wrong because the number of medical apparatuses is counted by humans. Further, it is desirable to reduce a burdensome procedure for a surgical operation on the medical staff members before and after the surgical operation.

In addition, the method of detecting the remnants with X-rays may impose on patients a high risk of radiation exposure, or having overlooked remnants resulting from a degraded imaging condition (radiographic condition) or inferior diagnostic reading skill. Moreover, this method is unable to detect the absence of the remnants or foreign materials in the body. Further, such determining tasks may consume labor of the staff members in the operating room, leading to elongation of the surgical operation or degradation of a patient's safety. Accordingly, it may be desirable in a social perspective to establish a system capable of preventing medical apparatuses or remnants being left in the body as well as reducing burdens of determining tasks by humans or X-ray.

Meanwhile, in a case where radio frequency identification (RFID) tags are attached to metallic products, an RFID reader may fail to perform wireless communications with the attached RFID tags due to the effect of metal.

Japanese Laid-open Patent Publication No. 2013-152352 (Patent Document 1) discloses a technique to prevent such wireless communications failure such as installing an insulating sheet or separating the RFID tags from the products. Further, Japanese Laid-open Patent Publication No. 2003-111772 (Patent Document 2) discloses a technique to attach a metallic RF tag in an opening part of a metallic medical apparatus.

However, in such a case, the RFID tag is externally attached to the metallic medical apparatus and is externally projected. Hence, this projection may interfere with the surgeon's work, or the original shape of the medical apparatus may be deformed or damaged due to the projection. Moreover, medical apparatuses such as surgical knives, surgical scissors, and surgical tweezers used in surgical operations are generally small, and an antenna shape of RFID tags attached to such medical apparatuses is small accordingly, which results in a short communications distance. Thus, it may be difficult to maintain a sufficiently long communications distance for detecting loss of medical apparatuses in surgical operating rooms.

RELATED ART DOCUMENTS

PATENT DOCUMENTS

Patent Document 1: Japanese Laid-open Patent Publication No. 2013-152352
Patent Document 2: Japanese Laid-open Patent Publication No. 2003-111772

Accordingly, it is a general object in one embodiment of the present invention to provide a medical apparatus with an IC chip capable of being identified by a communication function, and a medical apparatus management system capable of managing the medical apparatus with an IC chip that substantially obviate one or more problems caused by the limitations and disadvantages of the related art.

In one aspect of the embodiment, there is provided a medical apparatus with an IC chip. The medical apparatus includes a housing including a metallic part having a predetermined opening part; and an IC chip module having two conductive members, the conductive members being arranged on respective sides of the IC chip supplied with electricity via the conductive members. The IC chip module is attached such that the conductive members are arranged close to or on respective sides in a width direction of the predetermined opening part of the housing.

Other objects, features and advantages of the present invention will become more apparent from the following detailed description when read in conjunction with the accompanying drawings.

FIG. 1 is a diagram illustrating an example of a medical apparatus with an IC chip according to an embodiment;

FIGS. 2A and 2B are diagrams illustrating an example of an IC chip module attached to the medical apparatus according to the embodiment;

FIG. 3 is a diagram illustrating an example of a fabrication method of the medical apparatus with an IC chip according to the embodiment;

FIG. 4 is a diagram illustrating another example of a medical apparatus with an IC chip according to the embodiment;

FIG. 5 is a diagram illustrating another example of a medical apparatus with an IC chip according to the embodiment;

FIGS. 6A to 6D are diagrams illustrating a medical apparatus management system according to an embodiment;

FIG. 7 is a flowchart illustrating an example of a medical apparatus management system according to an embodiment; and

FIGS. 8A to 8D are diagrams illustrating an example of a log file according to an embodiment.

In the following, a description is given of embodiments of the present invention with reference to the accompanying drawings. Note that duplicated descriptions may be omitted by assigning identical reference numerals to those components having substantially the same functional configurations in the specification and the drawings of the present application.

INTRODUCTION

In general, medical apparatuses are managed by allowing medical staff members to count the number of medical apparatuses that come in and go out of an operating room before and after a surgical operation. However, in the above management, the counted number may be wrong because medical apparatuses are counted by people. Further, it is desirable to reduce a burdensome procedure for a surgical operation on the medical staff members before and after the surgical operation.

In the following, a description is given of an embodiment of a medical apparatus with an IC chip that is identifiable via a communications function. Subsequently, a description is given of a medical apparatus management system capable of managing the medical apparatus with the IC chip.

CONFIGURATION OF MEDICAL APPARATUS WITH IC CHIP

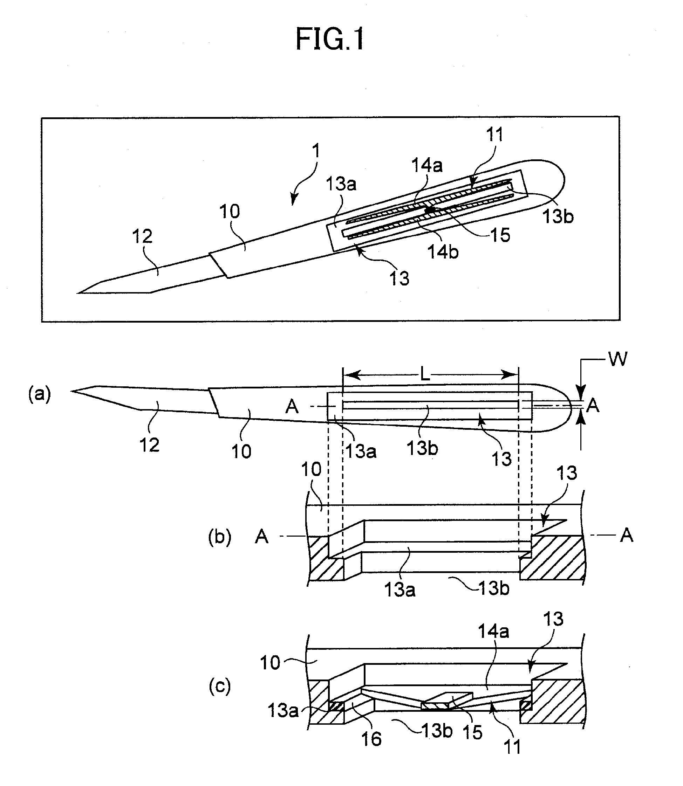

First, a configuration of a medical apparatus with an IC chip according to an embodiment is described with reference to FIG. 1. FIG. 1 illustrates an example of the medical apparatus with the IC chip according to the embodiment. The embodiment describes a surgical knife 1 that is given as an example of the medical apparatus to which an IC chip is attached. However, the medical apparatus according to the embodiment is not limited to the surgical knife 1, and may be various types of medical apparatuses including surgical scissors, tweezers, a surgical needle, forceps, screws, and the like. The medical apparatuses may be those used for surgical operations or those used for other medical applications.

The surgical knife 1, or a medical apparatus 1 according to the embodiment includes an IC chip module having a function to implement wireless communications. Specifically, the medical apparatus 1 includes a housing 10, an IC chip module 11, and a knife part 12. The housing 10 includes a metallic part. According to the embodiment, the housing 10 is formed of metal, and the overall housing 10 functions as a metallic part. The IC chip module 11 includes two conductive members 14a and 14b, and an IC chip 15. The conductive members 14a and 14b are formed of metal, and placed on two sides of the IC chip 15, respectively. The IC chip 15 is configured to receive electric supply from a later-described reader-writer via the conductive members 14a and 14b.

HOUSING: METALLIC PART

As illustrated in a diagram (a) of FIG. 1, the metallic part (i.e., the overall housing 10 in this example) includes an opening part 13. Examples of a material for the metallic part include steel, iron, copper, silver, and aluminum. Further, the metallic part may be formed of a composite material of plural materials selected from a group of conductive materials including steel, iron, copper, silver, and aluminum. The metallic part may form a part of the housing 10 or all of the housing 10. The metallic part may be formed of metal identical to that of the conductive members 14a and 14b, or a composite material including the metal identical to that of the conductive members 14a and 14b.

The opening part 13 includes a step part 13a, and a through hole 13b (a slit SLT) formed within the step part 13a. A perspective diagram (b) of FIG. 1 illustrates an A-A section of the diagram (a) of FIG. 1. A diagram (c) of FIG. 1 illustrates an IC chip module 11 placed on the step part 13a of the opening part 13 illustrated in the diagram (b) of FIG. 1. The IC chip module 11 is fixed to the step part 13a with adhesive or the like via a resin sheet 16 applied within the opening part 13. The IC chip module 11 may be fixed to the step part 13a using an insulating member such as resin adhesive instead of the resin sheet 16. In this case, the resin material is supplied and molded within the opening part 13 so as not to allow the IC chip module 11 to be detached from the opening part 13.

Note that the opening part 13 does not necessarily have the step part 13a. In this case, the opening part 13 may be the slit SLT that is the through hole 13b without having any step part. Further, the opening part 13 may have a tapered part inclined from an upper surface of the opening part 13 toward the through hole 13b in place of the step part 13a. Note also that, in a case where the opening part 13 is the through hole 13b without having the step part 13a, the through hole 13b may alternatively have an unpenetrated configuration. In this case, the opening part 13 may be an unpenetrated groove 13c (not illustrated). Further, the through hole 13b illustrated in FIG. 1 may be replaced with the groove 13c. That is, the opening 13 may also be composed of the step part 13a and the groove 13c.

An overall length L (see the diagram (a) of FIG. 1) of the slit SLT or the groove 13c of the opening part 13 with respect to a wavelength $\lambda$ of a radio wave used in wireless communications is may be obtained by L = $\lambda$/n, where L represents the overall length, $\lambda$ represents the wavelength of the radio wave, and n represents an integer of one or more. In this case, the highest voltage may be generated between two ends in a width direction of the opening part 13 that has received a radio wave. For example, the overall length L may be 160 mm when the frequency of the radio wave output from the reader-writer is 950 MHz, and the overall length L is 61 mm when the frequency of the radio wave is 2.45 GHz.

A width W (see the diagram (a) of FIG. 1) in the width direction of the slit SLT or the groove 13c of the opening part 13 is associated with a frequency width that allows an antenna to acquire a desired gain. That is, when the width in the width direction of the slit SLT or the groove 13c of the opening part 13 is gradually decreased, the frequency width that allows the antenna to acquire the desired gain may be decreased. By contrast, when the width in the width direction of the slit SLT or the groove 13c of the opening part 13 is gradually increased, the frequency width that allows the antenna to acquire the desired gain may be increased. However, when the width in the width direction of the slit SLT or the groove 13c of the opening part 13 is increased excessively, impedance may be increased and efficiency of the antenna may be decreased. Accordingly, it is preferable to increase the width in the width direction of the slit SLT or the groove 13c of the opening part 13 not to exceed a predetermined width.

In general, the slit SLT (the through hole 13b) is formed by punching a metallic mold, and optionally shaped by a secondary process. When the width W in the width direction of the slit SLT of the opening part 13 is too narrow, it may be difficult to obtain a desired width with a desired accuracy. Hence, the slit SLT may be formed by a laser process, which may, however, result in an increase in cost. Further, when the width W of the slit SLT is too narrow, antenna performance may be degraded by an exogenous material such as a metallic fragment caught by the through hole 13b. Hence, it may be preferable to form the slit SLT to have its width W in a range of 2 to 3 mm when the wavelength $\lambda$ of the radio wave used in wireless communications is 950 MHz. The groove 13c may also be formed to have its width W in the above range in a manner similar to similar to the slit SLT.

That is, the overall length L of the slit SLT or the groove 13c of the opening part 13 is represented by L=$\lambda$/n (n is an integer of one or more) when the wavelength of a wireless signal transmitted from the IC chip such as an RFID chip is represented by $\lambda$. The width W of the slit SLT or the groove 13c of the opening part 13 is set to have a length in a range of 7 to 9 mm when the wireless signal in UHF band is transmitted and received, and the width W of the slit SLT or the groove 13c of the opening part 13 is set to have a length in a range of 2 to 3 mm when the wireless signal in 2.45 GHz band is transmitted and received. In this case, the maximum voltage (the highest voltage) is generated between the two ends of the slit SLT or the groove 13c of the opening part 13 when n=2.

Further, the configuration of the slit SLT or the groove 13c is not limited to a linear shape. The slit SLT or the groove 13c may have a bent shape in so far as the overall length L of the slit SLT or the groove 13c is a predetermined length. For example, when the slit SLT has a configuration that is bent at two places, the slit SLT may have three parts (hereinafter called "first, second, and third slit parts S1, S2, and S3") having three lengths represented by L1, L2, and L3. In the following illustration, L1 represents a length of the first slit part S1, L2 represents a length of the second slit part S2, and L 3 represents a length of the third slit part S3. The second slit part S2 is located between the first slit part S1 and the second slit part S3. The second slit part S2 has a bent configuration, and is located adjacent to each of the slit parts S1 and S3, thereby forming a slit having a bent configuration. In this case, the slit SLT may be formed to satisfy the following equation. In the following equation, $\lambda$ represents a wavelength of a radio wave used in communications. For example, when the slit SLT is an H-shape, the slit SLT has two vertical lines (L1 and L2), and one horizontal line (L3) between the two vertical lines L1 and L2.

Overall length L of the slit SLT = L1 + L2 + L3 = $\lambda$/n (n is an integer of one or more) That is, even though the slit SLT or the groove 13c of the opening part 13 has a bent configuration, the overall length L of the slit SLT or the groove 13c of the opening part 13 may preferably be designed to have L = $\lambda$/n (n is an integer of one or more) so as to generate the highest voltage between the two ends in a width direction of the slit SLT or the groove 13c of the opening part 13.

IC CHIP MODULE

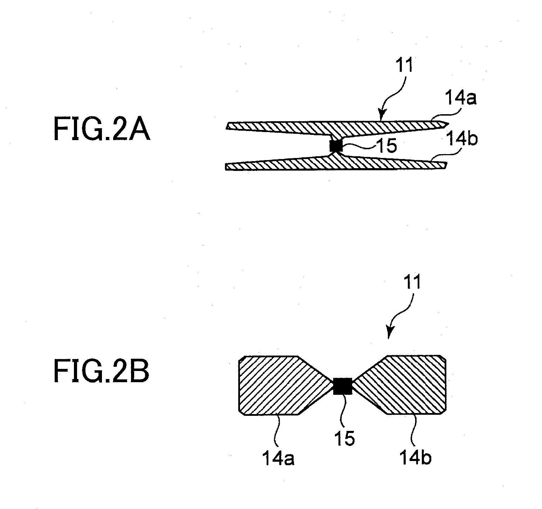

The IC chip module 11 may be an RFID tag capable of performing communications. As illustrated in FIGS. 2A and 2B, the IC chip module 11 includes conductive members 14a and 14b, and an IC chip 15. The conductive members 14a and 14b are formed of metal, and arranged on two sides of the IC chip 15, respectively. That is, the IC chip 15 is sandwiched between the conductive members 14a and 14b, and is configured to be operable when a potential difference between the conductive members 14a and 14b generated by a radio wave transmitted from the reader-writer causes an electric current to flow within the IC chip 15.

The IC chip module 11 may be an RFID tag that satisfies the following equation (relationship), where f represents the frequency of the radio wave used in wireless communications between the IC chip 15 and the reader-writer, Wa and V respectively represent an electromotive force and a voltage generated between two sides separated by a through hole 13b in a width direction of the opening part 13 when the IC chip module 11 receives the radio wave, S represents the areas of the conductive members 14a and 14b, d represents a thickness of an insulating sheet 16, $\varepsilon r$ represents a dielectric constant of the insulating sheet 16, $\varepsilon 0$ represents a dielectric constant of vacuum, and Wmin represents a minimum value of the power required for activating the IC chip 15.

$$\mathrm{Wmin} \leq \mathrm{Wa} - 4\pi f * S * \varepsilon 0 * \varepsilon r * V * 2/d$$

The shapes of the conductive members 14a and 14b are not limited to those illustrated in FIGS. 2A and 2B. For example, a length direction of the conductive members 14a and 14b is not restricted with respect to a length direction of the opening part 13. However, parts in the length direction of the conductive members 14a and 14b longer than the opening part 13 in the length direction will not be activated as electrodes. Hence, the length in the length direction of the conductive members 14a and 14b may preferably be shorter than the length in the length direction of the opening part 13. Further, in the above equation, the minimum value Wmin of the electricity required for activating the IC chip 15 may be reduced as the areas S of the conductive members 14a and 14b increase. The conductive members 14a and 14b may more advantageously function with respect to electricity supply when their contact areas are larger. Hence, the conductive members 14a and 14b may preferably have the maximum possible widths in the width direction of the opening part 13.

The communication frequency between the IC chip 15 and the reader-writer may generally be selected from the frequency bands such as 2.45 GHz, 5.8 GHz (microwave), and UHF (e.g., 950 MHZ). A base material for use in an antenna circuit board of the IC chip 15 is not specifically limited, and may be appropriately selected based on the purpose of its application. For example, the base material may be a rigid type such as paper phenol, glass epoxy and composite, a flexible type such as polyimide, polyester, polypropylene, polyethylene, polystyrene, nylon, polyethylene terephthalate (PET), paper and synthesized paper, or a combination of rigid and flexible types.

The thickness of the base material may preferably be in a range from 5 to 360 $\mu$m, and specifically preferably be in a range from 5 to 100 $\mu$m in view of processibility, operability, cost efficiency, and the like. Metallic foil for use in laminating the base material may be steel foil, iron foil, copper foil, silver foil, aluminum foil, and the like. Further, the metallic foil may be formed of a composite material of plural materials selected from a group of conductive materials including steel, iron, copper, silver, and aluminum. The aluminum foil may be preferable in view of processibility, operability, cost efficiency, and the like, and a preferable thickness range of the aluminum foil may be 2 to 50 $\mu$m. The shape of the aluminum foil is not specifically limited, and may be a square, a rectangle, a trapezoid, a circle, an oval, or a triangle.

The thickness (height) of the IC chip 15 may preferably be 200 $\mu$m or less, and may specifically preferably be in a range from 25 to 140 $\mu$m. Further, a protection film such as a polyimide film, a polyester film, or paper may be adhered to the IC chip 15 so as to protect the IC chip 15. The thickness of the protection film may preferably be in a range from 10 to 60 $\mu$m. The type of the IC chip 15 is not specifically limited, and any type of the IC chip may be appropriately selected based on the purpose of its application.

IMPLEMENTATION OF IC CHIP MODULE

The conductive members are attached to the metallic part of the surgical knife 1 to implement the IC chip module 11 in the surgical knife 1. The housing of some types of the medical apparatus may be formed of a material other than a metallic member such as resin. In such a case, the IC chip module 11 may be attached to the opening part 13 of the metallic part serving as a part of the housing.

As illustrated in the diagram (a) of FIG. 1, the IC chip module 11 is fixed to two opposite sides or respective areas close to the two opposite sides in the width direction of the opening part 13 within the opening part 13. That is, the IC chip module 11 is attached to the step part 13a of the opening part 13 such that the IC chip module 11 bridges the through hole 13b along the width direction of the opening part 13. Further, as illustrated in the diagram (c) of FIG. 1, the resin sheet 16 intervenes between the step part 13a of the opening part 13 and the conductive members 14a and 14b.

The IC chip module 11 may be attached to the opening part 13 using adhesive, or may be fixed to the opening part 13 using screws. To fix the IC chip 11 to the opening part 13 with screws, the screws may be inserted from screw holes formed in a holding plate to which the IC chip module 11 is attached, and the inserted screws may fix the holding plate to the step part 13a of the opening part 13. Further, the opening part 13 may be molded with a resin material, and the IC chip module 11 may be fixed to the opening part 13 by embedding the IC chip module 11 within the opening part 13. Note that the above-described method of attaching the IC chip module 11 to the metallic part of the medical apparatus is only one example, and is not limited to this example.

The adhesive for adhering the IC chip module 11 to the opening part 13 of the surgical knife 1 is not particularly specified, and any types of adhesive may be used insofar as the adhesive may fix the IC chip module 11 to the metallic part of the medical apparatus 1 (surgical knife 1). The resin used as the adhesive may be appropriately selected based on the purpose of its application. Examples of the resin material used as the adhesive include acrylic, polyethylene, polypropylene, polystyrene, polyvinyl alcohol, polyvinyl butyral, polyurethane, saturated polyester, unsaturated polyester, epoxy resin, phenolic resin, polycarbonate, and polyamide. Among these, resin cured by the application of heat, ultraviolet (UV) rays, and electron beams (EB) may appropriately be used, and thermosetting resin that is curable with a stiffening agent or moisture curable resin may be specifically preferable.

In addition, the opening part 13 provided with the IC chip module 11 may be filled with resin. In this case, the surface of the resin supplied on the IC chip module 11 may preferably be the same level (flat) as the surface of the housing 10 of the surgical knife 1. However, the surface of the resin may be lower than the surface of the housing 10 (recessed), or the surface of the resin may be higher than the surface of the housing 10 (projected). In any of the above cases, it may be preferable that the IC chip module 11 not project from the resin. Examples of a resin material filling in the opening part 13 include high-molecular compounds such as ceramic, polypropylene, and polyethylene, and may be appropriately selected based on the purpose of its application.

With the above configuration, the IC chip module 11 is fixed in the opening part 13 such that the IC chip module 11 bridges the through hole 13b of the opening part 13, and the conductive members 14a and 14b are separated from the metallic part (the step part 13a of the opening part 13 in this case) with an insulating material. Further, the IC chip module 11 is accommodated in the opening part 13. The IC chip 15 may be protected from damage due to shock by embedding the IC chip module 11 within the opening part 13.

Further, the shape of the opening part 13 is not particularly specified, and the opening part 13 may have any shape insofar as the opening part 13 may have sufficient space to accommodate the IC chip 15, and sufficient length L and width W required for performing communications. Moreover, the shape of the opening part 13 is not particularly specified with respect to the depth for implementing the IC chip module 11.

The above embodiment describes the surgical knife 1 with the IC chip module 11 as an example of the medical apparatus with the IC chip. Note that the IC chip 15 attached to the surgical knife 1 stores identification information for identifying the IC chip 15. The identification information stored by the IC chip 15 may be used as identification information of the medical apparatus with the IC chip 15. Hence, the medical apparatus with the IC chip according to the embodiment may be identifiable using a communications function.

FABRICATION METHOD OF MEDICAL APPARATUS WITH IC CHIP

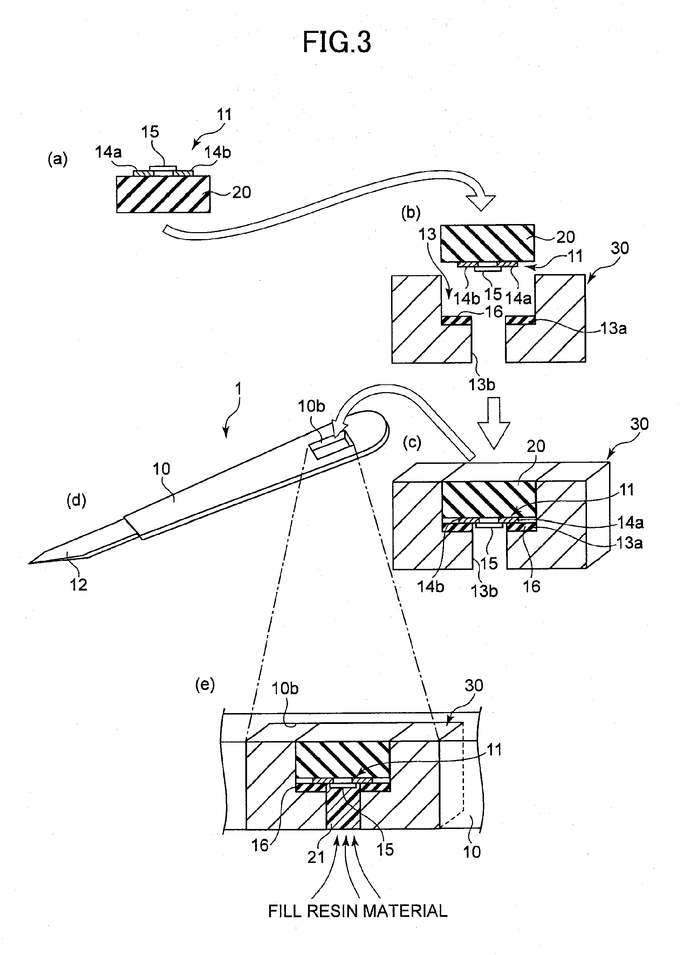

Next, a fabrication method of a medical apparatus with an IC chip according to an embodiment is described with reference to FIG. 3. FIG. 3 illustrates an example of the medical apparatus with the IC chip according to the embodiment. In this embodiment, the surgical knife 1 is given as an example of the medical apparatus, and a fabrication method of the surgical knife 1 with the IC chip 15 is illustrated.

First, the IC chip module 11 is adhered to a resin member 20 as illustrated in a diagram (a) of FIG. 3. The resin member 20 is formed in advance in a size so as to be appropriately fitted in the opening part 13.

Next, the resin member 20 having the downward directed IC chip module 11 is fitted in a metallic base 30 in which the opening part 13 is formed as illustrated in a diagram (b) of FIG. 3. The step part 13a of the opening part 13 is laminated with a resin sheet 16. Accordingly, when the resin member 20 is embedded in the metallic base 30, the IC chip module 11 is placed on the step part 13a via the insulating sheet 16, and the conductive members 14a and 14b may serve as electrodes of the IC chip 15 as illustrated in a diagram (c) of FIG. 3.

In this embodiment, a rectangular fitting part 10b is formed in the housing 10 of the surgical knife 1 as illustrated in a diagram (d) of FIG. 3. The metallic base 30 is formed in a size so as to be appropriately fitted in the fitting part 10b. Hence, when the metallic base 30 is fitted in the fitting part 10b, the metallic base 30 and the housing 10 form a flat surface of the metallic part of the surgical knife 1 with the IC chip 15 as illustrated in a diagram (e) of FIG. 3.

In this configuration, the resin material 21 is supplied and molded into the through hole 13b from a rear surface of the metallic base 30 penetrating the housing 10 such that the IC chip module 11 is fixed to the opening part 13 and not detached from the opening part 13 as illustrated in the diagram (e) of FIG. 3.

MODIFICATION

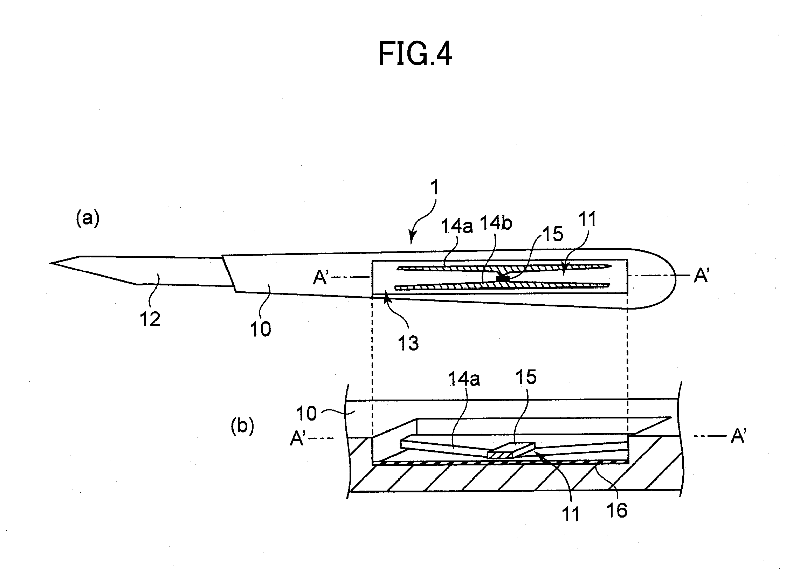

Note that as a modification of the medical apparatus with IC chip according to the embodiment, a configuration having a groove serving as the opening part 13 without having the through hole is depicted in FIG. 4. A perspective diagram (b) of FIG. 4 illustrates an A'-A' section of the diagram (a) of FIG. 4. In this case, the IC chip module 11 is fixed to the opening part 13 with adhesive or the like via the resin sheet 16 that is laminated within the opening part 13. In this case, a resin material may be supplied and molded within the opening part 13 so as not to allow the IC chip module 11 to be detached from the opening part 13.

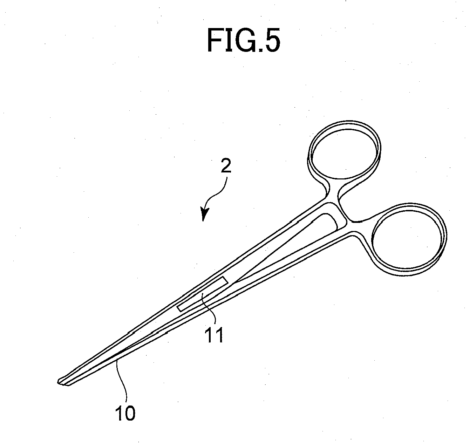

In the above, a description is given of the method of attaching the IC chip 11 to the metallic part of the medical apparatus. The fabrication method described above is not limited to a method of attaching the IC chip module 11 to the surgical knife 1, and may similarly be applicable to a fabrication method of surgical apparatuses other than the surgical knife 1. The fabrication method described above may be applied to a method of attaching the IC chip module 11 to a metallic part of the housing 10 of surgical scissors as illustrated in FIG. 5.

MEDICAL APPARATUS MANAGEMENT SYSTEM

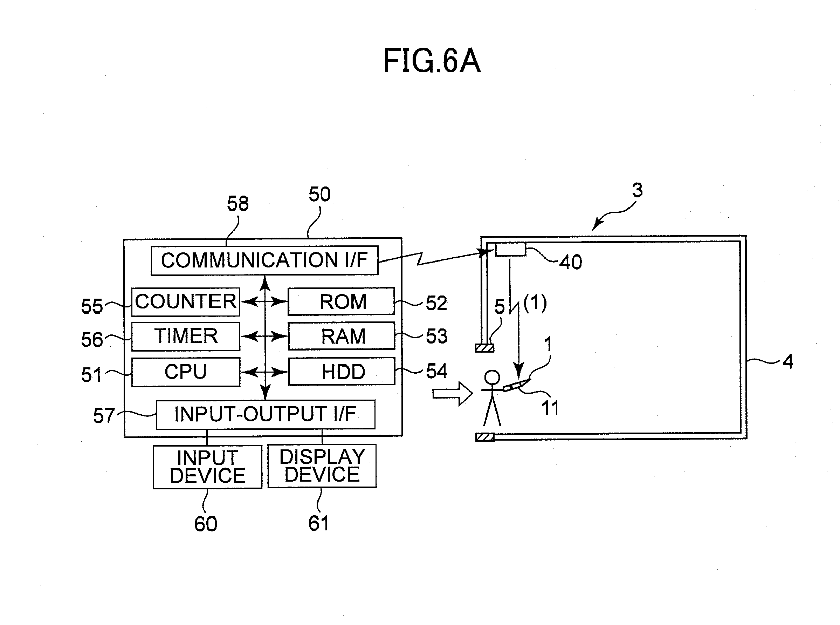

Next, a medical apparatus management system according to an embodiment is described with reference to FIGS. 6A to 6D. FIGS. 6A to 6D illustrate an example of the medical apparatus management system according to the embodiment. In this embodiment, a medical apparatus management system 3 manages medical apparatuses used in an operating room 4.

The medical apparatus management system 3 includes medical apparatuses (a surgical knife 1 is depicted as an example of the medical apparatus in FIGS. 6A to 6D), a reader-writer 40, and a personal computer (PC) 50. The surgical knife 1 is provided with an IC chip module 11. Note that the IC chip module 11 is attached not only to the surgical knife 1, but also all the medical apparatuses managed by the medical apparatus management system 3 are provided with IC chip modules 11.

When the frequency band is a long wave band or a short wave band, a voltage is induced in the RFID tag due to electromagnetic induction between a transmission antenna coil of the reader-writer and an antenna coil of the RFID tag, and this voltage activates the IC chip such that the IC chip becomes communicative. Thus, in the communications with an electromagnetic induction type, the RFID tag is operable only within the induction field, and hence, the communications distance may be short such as several centimeters.

On the other hand, the IC chip module 11 used in the embodiment may be driven by electric energy received from the reader-writer 40. Further, the frequency band for use in the wireless communications may be a UHF band and a microwave band. Hence, since the medical apparatus management system 3 performs communications with a radio frequency communications type, the communications distance may be long such as 1 to 8 meters.

Accordingly, in this embodiment, since the reader-writer 40 is placed on a ceiling located above a gate 5 in the operating room 4, the IC chip module 11 attached to the surgical knife 1 may detect a radio wave transmitted from the reader-writer 40 when a person and the like passes through the gate 5. Hence, the IC chip module 11 may be able to receive electric energy and transmit detected information including identification information to the reader-writer 40. Note that the operating room 4 is an example of a predetermined room in which medical apparatuses are involved in medical practice, and the predetermined room includes a consultation room, a medical examination room, and the like. The gate 5 is at a doorway of the predetermined room in which medical staff members engage in medical practice. The gate 5 includes sensors configured to detect a person's comings and goings in order to detect whether one or more persons come in the operating room or go out of the operating room.

The PC 50 includes a central processing unit (CPU) 51, a read only memory (ROM) 52, a random access memory (RAM) 53, a hard disk drive (HDD) 54, a counter 55, a timer 56, an input-output interface (I/F) 57, and a communications I/F 58 that are connected to one another via a bus.

The ROM 52 is non-volatile semiconductor memory (a storage device) configured to retain internal data when power supply is turned off. The ROM 52 stores programs and data such as those for OS settings or network settings. The RAM 53 is volatile semiconductor memory configured to temporarily store programs and data. The HDD 54 serves as a non-volatile storage device configured to store programs and data. Examples of the stored programs and data include an operating system (OS) serving as basic software that is configured to control each of apparatuses as a whole, application software that is configured to provide various functions on the OS, and the like.

The CPU is a processor configured to implement control over the apparatuses or functions of the apparatuses by loading programs and data in the RAM 53 from the storage device (e.g., the HDD 54) to execute processes including the later-described medical apparatus management process.

The counter 55 is configured to count the number of medical apparatuses present in the operating room. The timer 56 is configured to measure time. The input-output interface (I/F) 57 serves as an interface configured to acquire input data in accordance with operations of the input device 60 such as a keyboard or a mouse to display necessary data on a screen of the display device 61. The communications I/F 58 serves as an interface configured to perform communications with external apparatuses such as the reader-writer 40 via a network.

Note that the PC 50 is an example of an information processing apparatus configured to manage medical apparatuses with IC chips that have passed through the gate. The information processing apparatus according to an embodiment is not limited to the PC 50, and may be any apparatuses such as tablet-type terminals insofar as the apparatuses have a communications function and an information processing function. The information processing apparatus is configured to manage medical apparatuses with IC chips 15 inside the room based on identification information of the medical apparatuses with IC chips 50 received by the reader-writer 40. The information processing apparatus is configured to manage medical apparatuses with IC chips 15 for each of the types of the medical apparatuses inside the room based on identification information and type information of the medical apparatuses with IC chips 50 received by the reader-writer 40.

MEDICAL APPARATUS MANAGEMENT PROCESS

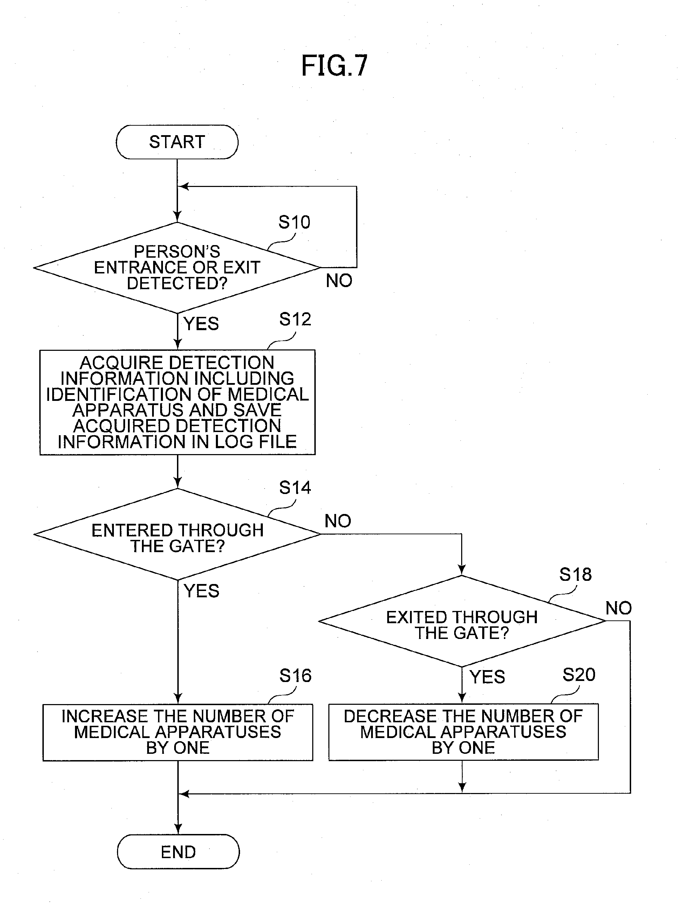

Next, a medical apparatus management process executed by the PC 50 according to an embodiment is illustrated with reference to FIG. 7. FIG. 7 is a flowchart illustrating an example of a medical apparatus management system according to an embodiment. In this embodiment, the medical apparatus management system 3 is configured to manage medical apparatuses used in the operating room 4.

When the medical apparatus management process starts, the CPU 51 of the PC 50 determines whether a sensor attached to the gate 5 detects a person's coming in or going out of the operating room 4 (step S10). When the sensor does not detect a person's coming or going, the CPU 51 of the PC 50 repeats a process of step S10. When the sensor has detected a person's coming or going, the CPU 51 acquires detection information including identification information of the medical apparatus with the IC chip that has passed through the gate 5 via the reader-writer 40, and saves the acquired detection information in a log file (step S12).

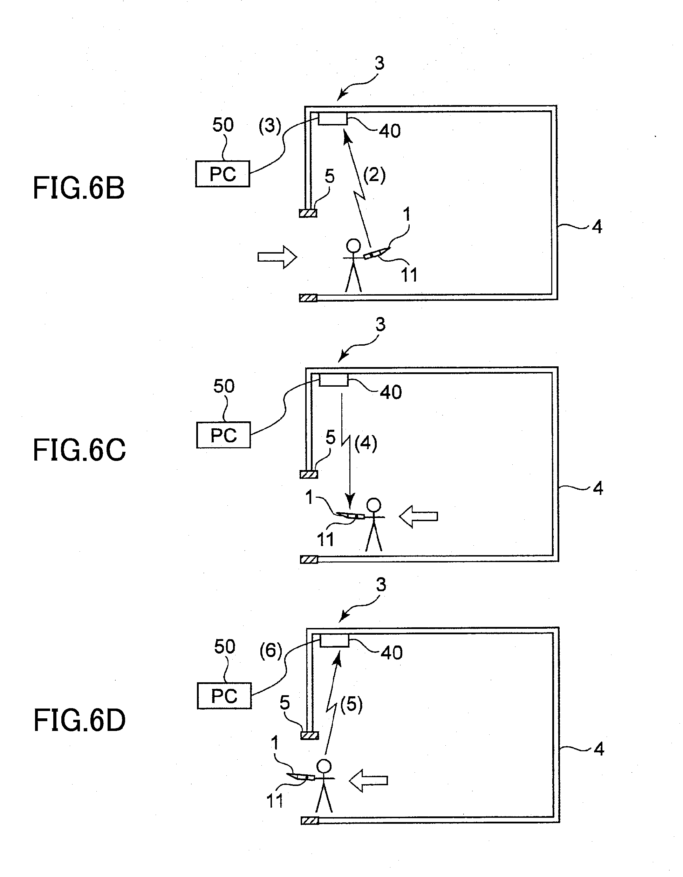

For example, when the surgical knife 1 with the IC chip 15 is brought into the operating room 4 via the gate 5, the IC chip module 11 of the surgical knife 1 receives a radio wave transmitted from the reader-writer 40 (see (1) of FIG. 6A). The IC chip module 11 transmits detection information including at least identification information of itself to the reader-writer 40 using supplied electric energy (see (2) of FIG. 6B). The detection information preferably includes information about a type (type information) of the medical apparatus. The transmitted detection information is read by the reader-writer 40.

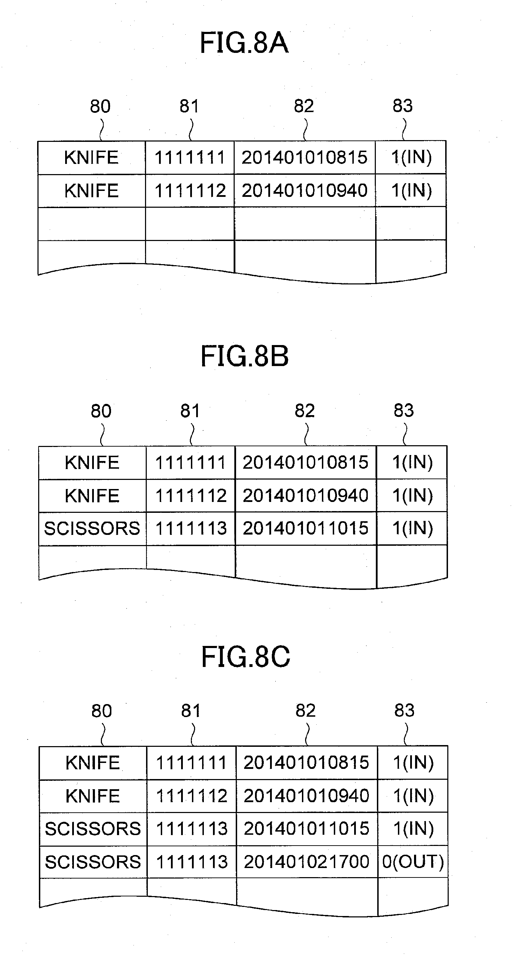

The CPU 51 acquires the detection information read by the reader-writer 40 via the communications I/F 58, and saves the acquired detection information in the RAM 53 or the HDD 54 (see (3) of FIG. 6B). FIGS. 8A to 8D illustrate examples of a log file illustrating history information of medical apparatuses coming in or going out of the operating room 4 saved in the RAM 53 or HDD 54.

Log file records type information 80 of each medical apparatus, identification information 81 (i.e., identification information of IC chip 15) of the medical apparatus, time information 82 acquired at a time at which the detection information is acquired, and location information 83 of the medical apparatus (1: IN (indoor), 0: OUT (outdoor)). In this embodiment, the type information 80 of the medical apparatus, the identification information 81 of the medical apparatus, and the location information 83 of the medical apparatus are included in the detection information transmitted from the IC chip 15 attached to the surgical knife 1.

As described above, the IC chip 15 receives radio waves transmitted from the reader-writer 40, and transmits the detection information at least including identification information of itself when entering a communications area of the reader-writer 40. Hence, the PC 50 may be able to acquire the detection information via the reader-writer 40 and generate a log file for managing the medical apparatuses.

Next, referring back to FIG. 7, the CPU 51 determines whether the surgical knife 1 has entered via the gate 5 (step S14). When the location information 83 of the medical apparatus in the log file indicates 1 (IN), the CPU 51 determines that the surgical knife 1 has entered via the gate 5, and the counter 55 increments the number of medical apparatuses by one (step S16).

For example, as illustrated in FIG. 8A, when the log file records history information of entrance or exit of two medical apparatuses, location information 83 of the two medical apparatuses (medical knives 1) are all 1 (IN), and the counter 55 calculates the number of the surgical knives 1 as "2". As illustrated in FIG. 8B, when the log file records history information of entrance or exit of three medical apparatuses, the location information 83 of the two surgical knives 1 and location information 83 of a pair of surgical scissors 2 are both 1 (IN). In this case, the counter 55 calculates the number of the surgical knives 1 as "2", and the number of pairs of surgical scissors 2 as "1".

In the following, an illustration is given of a case where the pair of surgical scissors 2 with an IC chip is taken out of the operating room 4 via the gate 5. Initially the CPU 51 determines whether the sensor attached to the gate 5 detects a person's coming in or going out of the operating room 4 (step S10). When the sensor does not detect the people's coming or going, the CPU 51 of the PC 50 repeats a process of step S10. When the sensor has detected the people's coming or going, the CPU 51 acquires detection information including identification information of the medical apparatus with the IC chip that has passed through the gate 5 via the reader-writer 40, and saves the acquired detection information in a log file (step S12).

For example, when the pair of surgical scissors 2 with an IC chip is taken out of the operating room 4 via the gate 5, the IC chip module 11 of the pair of surgical scissors 2 receives a radio wave transmitted from the reader-writer 40 (see (4) of FIG. 6C). The IC chip module 11 transmits detection information including at least identification information of itself to the reader-writer 40 using supplied electric energy (see (5) of FIG. 6D). The detection information preferably includes information about a type (type information) of the medical apparatus. The transmitted detection information is read by the reader-writer 40.

The CPU 51 acquires the detection information read by the reader-writer 40 via the communications I/F 58, and saves the acquired detection information in the RAM 53 or the HDD 54 (see (6) of FIG. 6D). In FIG. 8C, exit history information, for the surgical scissors 2 recorded on the third line, is recorded on the fourth line. Thus, location information 83 on the fourth line indicates 0 (OUT).

Next, referring back to FIG. 7, the CPU 51 determines whether the surgical scissors 2 have entered via the gate 5 (step S14). In this case, the surgical scissors 2 are taken out of the operating room 4 via the gate 5. Thus, the CPU 51 determines this case as "NO" in step S14, and proceeds with step S18. In step S18, the CPU 51 determines that the surgical knife 1 is taken out of the operating room 4 via the gate 5 based on the location information 83 of the scissors 2 on the fourth line of the log file in FIG. 8C. The counter 55 reduces the number of pairs of surgical scissors 2 by one (step S20). As a result, the counter calculates the number of pairs of surgical scissors as "0". At this time, the counter 55 calculates the number of surgical knives 1 as "2". In step S18, when the CPU 51 determines the case as "No", no response or an error may be output.

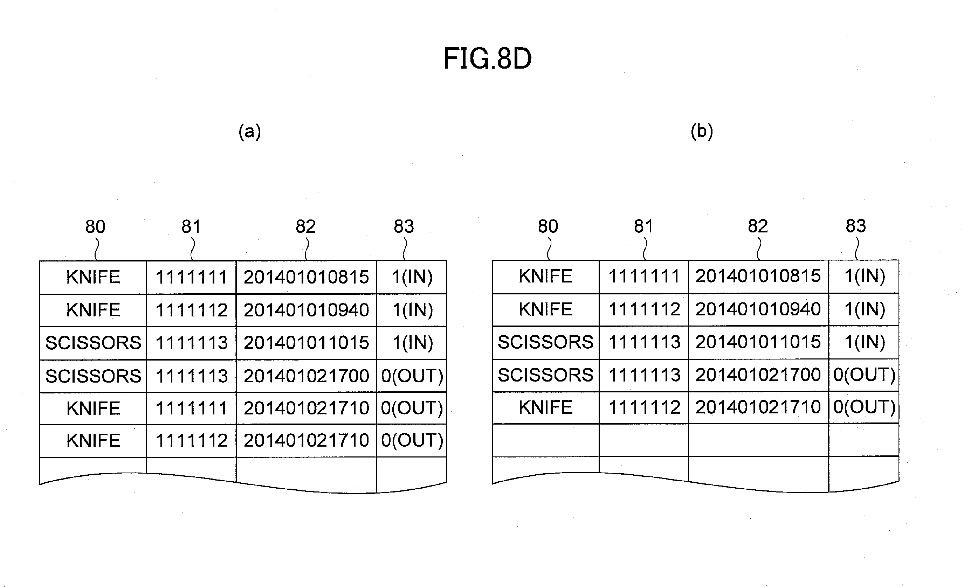

When a log file as illustrated in (a) of FIG. 8D is generated as a result of the medical apparatus management process according to the embodiment based on the entrance or exit of the medical apparatuses illustrated in FIG. 7, the counter 55 calculates the number of surgical knives 1 and the number of pairs of scissors 2 both as "0". The CPU 51 may be able to determine that there is no medical apparatus remaining in the operating room 4.

On the other hand, when a log file illustrated (b) of FIG. 8D is generated, the counter 55 calculates the number of surgical knives 1 as "1" and the number of pairs of scissors 2 as "0". In this case, the CPU 51 may be able to determine that the surgical knife 1 having identification information "1111111" remains in the operating room 4.

The illustration is given above of the medical apparatus with the IC chip and the medical apparatus management system according to the embodiment. According to the above-described embodiments, it may be possible to provide a medical apparatus with an IC chip capable of being identified using a communications function, and a medical apparatus management system capable of managing such a medical apparatus.

Note that the information saved in the log file generated in the above embodiments may be displayed on the display device 61. Further, the reader-writer in the embodiments may be an antenna or a transmitter-receiver embedded in a surgical operation bed, or an antenna or a transmitter-receiver embedded in a medical apparatus table device. Further, the reader-writer in the embodiments is not particularly specified, and the reader-writer may include two or more antennas or transmitter-receivers for eliminating blind spots.

As illustrated above, the medical apparatus according to the embodiments is provided with a readable-writable IC chip module for wirelessly identifying the medical apparatus via wireless communications. Accordingly, the medical apparatuses with IC chips may be incorporated in the medical apparatus management system including the reader-writer and the information processing apparatus so as to save real-time history information of the presence (coming in) or absence (going out) of the medical apparatuses in the operating room before, after and during the operation. Then, the location of the medical apparatuses may be monitored based on the log file storing history information at any time with the information processing apparatus. Further, burdens associated with management of the medical apparatuses may be significantly decreased as well as securing the safety of the patients. In addition, even when a medical accident with remnants occurs by any chance, the remnants may be collected using the IC chip module.

Further, antenna scanners may be included for easily detecting the locations of the remnants. Further, since entrance or exit of the medical apparatuses with IC chips may be determined in real-time by introducing the IC chip module, it may be possible to easily detect the medical apparatuses, the time of their use, and their locations during the surgical operations. Hence, even when any one of the medical apparatuses is accidentally lost, there may remain some detectable information or clue for discovering the lost medical apparatus, and hence, the medical apparatus that has been lost may be safely collected. Further, when complicated medial apparatuses are prepared for surgical operations, types of the medical apparatuses may be managed in advance, which may facilitate the management associated with sterilization treatment.

The medical apparatus with the IC chip and the medical apparatus management system are described above with the embodiments; however, the present invention is not limited to these embodiments described above. Various alteration and modification may be made within the scope of the claims.

For example, as a material for molding the surface or rear surface of the opening part formed in the medical apparatus of the embodiments, a predetermined polymer (2-methacryloyloxyethyl phosphorylcholine) or hydroxyapatite (a kind of calcium phosphate) may be embedded. The above-described materials will not affect radio communications and may be preferable because such materials will not allow the IC chip to extrude to interfere with the surgeon's work.

In the above embodiments, the surgical knife and the surgical scissors are given as examples of the medical apparatus with an IC chip. However, the medical apparatus with an IC chip is not limited to these examples. Other medical apparatuses such as surgical scissors, surgical tweezers, medical suture needles, forceps, surgical crews, and the like that may remain in the body of the patient during surgical operations may be include an IC chip so as to exhibit the same effects as the surgical knife 1 or surgical scissors 2 of the embodiments.

According to an aspect of the embodiments, it may be possible to provide a medical apparatus with an IC chip capable of being identified using a communications function, and a medical apparatus management system capable of managing such a medical apparatus.

The present invention is not limited to the specifically disclosed embodiments, and variations and modifications may be made without departing from the scope of the present invention.

**Claims**

1. A medical apparatus (1) with an IC chip (15), the medical apparatus comprising:

   a housing (10) including a metallic part having a predetermined opening part; and
   an IC chip module (11) having two conductive members (14a, 14b), the conductive members being arranged on respective sides of the IC chip supplied with electricity via the conductive members (14a, 14b), wherein the IC chip module (11) is attached such that the conductive members (14a, 14b) are arranged close to or on respective sides in a width direction of the predetermined opening part of the housing.

2. The medical apparatus as claimed in claim 1, wherein
   the predetermined opening part includes a through hole.

3. The medical apparatus as claimed in claim 1 or 2, wherein
   the predetermined opening part includes a step part, and
   the IC chip module fixed to the step part via a resin material within the predetermined opening part.

4. The medical apparatus as claimed in any one of claims 1 to 3, wherein
   a resin material is molded within the predetermined opening part such that the IC chip module is embedded within the predetermined opening part.

5. The medical apparatus as claimed in any one of claims 1 to 4, wherein
   the metallic part is formed of a metal identical to that of the conductive members, or a composite material including the metal identical to that of the conductive members.

6. The medical apparatus as claimed in any one of claims 1 to 5, wherein
   an overall length in a length direction of the predetermined opening part with respect to a wavelength of a radio wave transmitted from a reader-writer that is wirelessly in communications with the IC chip module is obtained by $L = \lambda/n$, wherein L represents the overall length of the predetermined opening part, $\lambda$ represents the wavelength of the radio wave transmitted from the reader-writer in wireless communications with the IC chip module, and n represents an integer of one or more.

7. A medical apparatus management system comprising:

   an information processing apparatus;
   a medical apparatus with an IC chip; and
   a reader-writer configured to wirelessly communicate with the medical apparatus, wherein
   the medical apparatus includes
   a housing including a metallic part having a predetermined opening part; and
   an IC chip module having two conductive members, and configured to wirelessly communicate with the reader-writer, the conductive members being arranged on respective sides of the IC chip supplied with electricity via the conductive members, wherein

the IC chip module is attached such that the conductive members are arranged close to or on respective sides in a width direction of the predetermined opening part of the housing, and wherein

the IC chip receives from the reader-writer electricity to transmit detection information including identification information of the medical apparatus to the reader-writer, and

the information processing apparatus manages the medical apparatus based on the identification information included in the detection information received by the reader-writer.

8. The medical apparatus management system as claimed in claim 7, wherein

the detection information includes the identification information and type information of the medical apparatus, and when the medical apparatus is carried inside or carried outside through a gate located at a doorway of a predetermined room, the information processing apparatus manages one or more of the medical apparatuses of one or more types of the medical apparatuses, based on the identification information and the type information of the medical apparatus included in the detection information received by the reader-writer.

# FIG.1

(a)

(b)

(c)

FIG.2A

11
14a
14b
15

FIG.2B

11
14a
15
14b

# FIG.3

(a)

(b)

(c)

(d)

(e)

FILL RESIN MATERIAL

FIG.4

# FIG.5

FIG.6A

FIG.6B

FIG.6C

FIG.6D

# FIG.7

```
                    ┌─────────────┐
                    │    START    │
                    └─────────────┘
                           │
            ┌──────────────┤◄─────────────────┐
            │              ▼          S10      │
            │         ╱PERSON'S╲               │
            │        ╱ ENTRANCE OR EXIT ╲  NO  │
            │        ╲ DETECTED?       ╱───────┘
            │         ╲               ╱
            │              │ YES        S12
            │    ┌──────────────────────┐
            │    │   ACQUIRE DETECTION   │
            │    │ INFORMATION INCLUDING │
            │    │ IDENTIFICATION OF     │
            │    │ MEDICAL APPARATUS AND │
            │    │ SAVE ACQUIRED         │
            │    │ DETECTION INFORMATION │
            │    │ IN LOG FILE           │
            │    └──────────────────────┘
```

ACQUIRE DETECTION INFORMATION INCLUDING IDENTIFICATION OF MEDICAL APPARATUS AND SAVE ACQUIRED DETECTION INFORMATION IN LOG FILE

S14 — ENTERED THROUGH THE GATE?

S18 — EXITED THROUGH THE GATE?

YES — S16 — INCREASE THE NUMBER OF MEDICAL APPARATUSES BY ONE

YES — S20 — DECREASE THE NUMBER OF MEDICAL APPARATUSES BY ONE

END

# FIG.8A

| 80 | 81 | 82 | 83 |
| --- | --- | --- | --- |
| KNIFE | 1111111 | 201401010815 | 1(IN) |
| KNIFE | 1111112 | 201401010940 | 1(IN) |
|  |  |  |  |
|  |  |  |  |

# FIG.8B

| 80 | 81 | 82 | 83 |
| --- | --- | --- | --- |
| KNIFE | 1111111 | 201401010815 | 1(IN) |
| KNIFE | 1111112 | 201401010940 | 1(IN) |
| SCISSORS | 1111113 | 201401011015 | 1(IN) |
|  |  |  |  |

# FIG.8C

| 80 | 81 | 82 | 83 |
| --- | --- | --- | --- |
| KNIFE | 1111111 | 201401010815 | 1(IN) |
| KNIFE | 1111112 | 201401010940 | 1(IN) |
| SCISSORS | 1111113 | 201401011015 | 1(IN) |
| SCISSORS | 1111113 | 201401021700 | 0(OUT) |
|  |  |  |  |

# FIG.8D

(a)

(b)

EP 2 929 853 A2

| 80 | 81 | 82 | 83 |
|---|---|---|---|
| KNIFE | 1111111 | 201401010815 | 1(IN) |
| KNIFE | 1111112 | 201401010940 | 1(IN) |
| SCISSORS | 1111113 | 201401011015 | 1(IN) |
| SCISSORS | 1111113 | 201401021700 | 0(OUT) |
| KNIFE | 1111111 | 201401021710 | 0(OUT) |
| KNIFE | 1111112 | 201401021710 | 0(OUT) |
| | | | |

| 80 | 81 | 82 | 83 |
|---|---|---|---|
| KNIFE | 1111111 | 201401010815 | 1(IN) |
| KNIFE | 1111112 | 201401010940 | 1(IN) |
| SCISSORS | 1111113 | 201401011015 | 1(IN) |
| SCISSORS | 1111113 | 201401021700 | 0(OUT) |
| KNIFE | 1111112 | 201401021710 | 0(OUT) |
| | | | |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2013152352 A

- JP 2003111772 A